Europäisches Patentamt

European Patent Office

Office européen des brevets

(19)

(11) Publication number: **0 305 063**

**A2**

(12) **EUROPEAN PATENT APPLICATION**

(21) Application number: **88307136.7**

(22) Date of filing: **02.08.88**

(51) Int. Cl.⁴: **C12P 21/00 , C12N 9/00**

(30) Priority: **04.08.87 JP 195087/87**

(43) Date of publication of application:
**01.03.89 Bulletin 89/09**

(84) Designated Contracting States:
**CH DE FR GB IT LI**

(71) Applicant: **Kissei Pharmaceutical Co Ltd
No. 19-48 Yoshino
Matsumoto-shi Nagano(JP)**

(72) Inventor: **Okamoto, Hiroshi
No. 205, 15-3 Tsunogoro 2-chome
Sendai-shi Miyagi(JP)**

(74) Representative: **Pearce, Anthony Richmond et
al
MARKS & CLERK Alpha Tower Suffolk Street
Queensway Birmingham B1 1TT(GB)**

(54) **A process for production of c-terminal alpha-amide peptides.**

(57) A process which biologically matures or modifies peptide hormone precursors and neuropeptide precursors to des-glycine α-amide peptide hormones or neuropeptides by cleavage of N-C bond in the glycine molecule.

Fig. 1

Fraction Number

EP 0 305 063 A2

## A PROCESS FOR PRODUCTION OF C-TERMINAL α-AMIDE PEPTIDES

### FIELD OF THE INVENTION

The present invention relates to a process which matures or modifies peptides having C-terminal glycine to des-glycine α-amide peptides by cleavage of N-C bond in the glycine molecule. More detail, the present invention relates to a process which biologically matures or modifies peptide hormone precursors and neuropeptide precursors to des-glycine α-amide peptide hormones or neuropeptides by cleavage of N-C bond in the glycine molecule.

### BACKGROUND OF THE INVENTION

Peptidylglycine α-amidating activity, which catalyzes the oxidation of C-terminally glycine-extended peptides to des-glycine α-amide peptides, was first detected in porcine pituitary by Brudbury et al. using a synthetic tripeptide, D-Tyr-Val-Gly, as a substrate (Nature 298, 686-688, 1982). In 1983, Eipper et al. also detected a similar activity in rat pituitary and found that it was stimulated by the addition of copper ion and ascorbic acid and that it required the presence of molecular oxygen, leading to the proposal that it be named peptidylglycine α-amidating monooxygenase (J. Biol. Chem. 259, 6385-6392 1984). Since then, the occurrence of the copper- and ascorbate-stimulated and oxygen-requiring α-amidating activity in various tissue such as the brain (Proc. Natl. Acad. Sci. 81, 3228-3232, 1984), the exocrine glands including the parotid gland (Proc. Natl. Acad. Sci. 83, 3297-3301, 1986) and the neonatal pancreas (Biochem. Biophys. Res. Commun 138, 179-184, 1986) has been well documented. The α-amidating activity appears to have a quite broad substrate specificity; a wide variety of glycine-extended peptides are converted to des-glycine α-amide peptides (Biochem. Biophys. Res. Commun. 112, 372-377, 1983). Current data suggest that the activities seem to be involved in vivo in the C-terminal α-amide formation of bioactive polypeptides, for most of which, the presence of the amide group is essential to bring about the optimal physiological functions (J. Biol. Chem 259, 13041-13048, 1984., J. Biol. Chem. 260, 16224-16231, 1985., J. Biol. Chem. 261, 11938-11941, 1986). Attempts have been made to purify the α-amidating enzyme from several tissues including bovine (J. Biol. Chem. 261, 1815-1822, 1986) and porcine (Endocrinol. 118, 2262- 2267, 1986) pituitaries and Xenopus skin (Biochem. Biophys Res. Commun 137, 984-991, 1986); the preparations appeared to be near homogenous, although the yields were relatively low. Therefore, problems to be solved are the development of the purification process to obtain homogenous enzyme and of process to restore the enzyme activity to the original level.

Nowadays attempts have been made to produce bioactive polypeptides by DNA recombinant technics and cell culture technics. However, most of produced polypeptides, if not all, must be matured or modified to C-terminal α-amide peptides to function as fully active peptides. However α-amidation by chemical methods is quite difficult and the economical cost is extremely high. On the other hand, efforts have been made to mature or modify by biochemical method employing α-amidating enzyme, however, since the more the enzyme is purified, the more the activity is lowered as described above, the efficient biochemical process for α-amidation has not been yet established.

### SUMMARY OF THE INVENTION

An object of the present invention is to provide a novel process for production of C-terminal α-amide peptides.

Another object of the present invention is to provide a process which biologically matures or modifies peptide hormone precursors and neuropeptide precursors to des-glycine α-amide peptide hormones or neuropeptides by cleavage of N-C bond in the glycine molecule.

Other objects, features and advantages of the present invention will be apparent from the following description of the invention.

## BRIEF DESCRIPTION OF THE DRAWINGS

Figure 1 is a graph showing the elution profile of rat brain α-amidating activity from Sephacryl S-200 gel chromatography as in Example 3 hereinafter described.

Figure 2 is a graph showing the effect on the α-amidating activity of the Sephacryl S-200 fraction, obtained by adding various amounts of the boiled supernatant as in Example 4 hereinafter described.

## DETAILED DESCRIPTION OF THE INVENTION

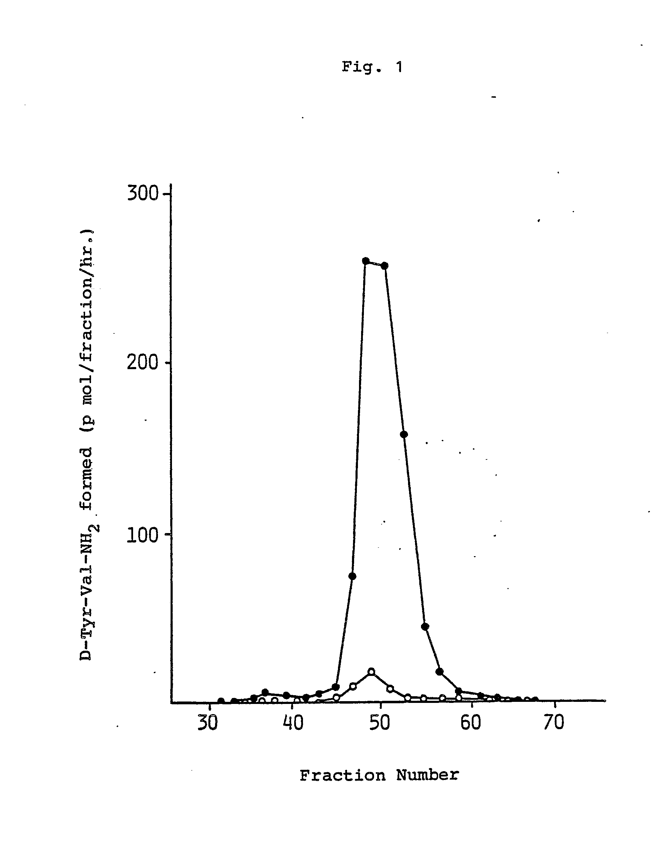

In Figure 1, the ordicate shows the amounts of D-Try-Val-NH₂ formed (p mol/fraction/hr., —— • —— and —— • ——), and the abscissa indicates the fraction numbers.

The α-amidating activities were assayed using 25 μl of each faction under the conditions without (—— ○ ——) or with (——• ——) 10 μl of the boiled supernatant prepared from ammonium sulfate fraction.

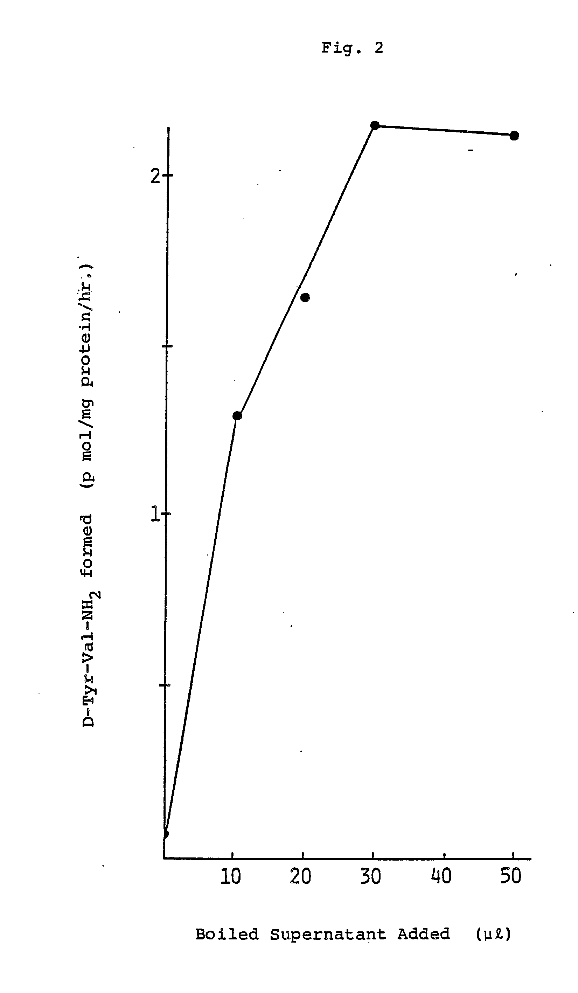

In Figure 2, the ordicates indecates the amount of D-Try-Val-NH₂ formed (p mol/protein mg/hr.) and the abscissa indicates the volume of the boiled supernatant added (μl). The assay mixtures contained 50 μl (6.8 μl as protein) of the combined Sephacryl S-200 fraction and the indicated amounts of the boiled supernatant.

## DETAILED DESCRIPTION OF THE INVENTION

The present inventors found that α-amidating enzyme, when purified or partially purified, showed an extremely lower, often only marginal, activity than that expected from original activity, but that the addition of other factor, which is an enzymatic cofactor or prepared from tissue homogenate, was able to restore the α-amidating activity of the purified enzyme almost to the original level. In another words, the present inventors found that the addition of factor which is partially purified from tissue homogenate by salting out and dialysis was able to restore the purified α-amidating enzyme activity to the original level, and that the restoration was also accomplished by the addition of common enzymatic cofactor like a pyrroloquinolinequinone.

The restoring factor was prepared from tissue homogenate as described below.

Rat brains were homogenized in suitable buffer and centrifuged. The supernatant was fractionated with solid ammonium sulfate and the precipitate obtained was suspended in homogenizing buffer and then dialyzed. Insoluble materials were removed and the supernatant was boiled for a few minutes. However, the boiling, a simple method for purification, was not absolutely necessary and treatment with proteinase K or trypsin was also available for purification.

The fraction, prepared from tissue homogenate as described so far, contained a factor, referred to as the α-amidation restoring factor that was able to restore the inevitable loss of α-amidating activities through purification. This restoring factor appeared quite stable; it could be kept at -20°C over 3 months without loss of the restoring activity and was not affected by incubation with up to 1 mM H₂O₂ for 1 hr.

It was hardly extracted in organic solvents such as ethanol or chloroform and could not be filtered through a Centricut membrane cutting molecules of over 10,000 daltons.

This factor itself·had no α-amidation activity. Furthermore, the restoring activities were not destroyed by treatment with trypsin or proteinase K. The restoring system, wherein α-amidating enzyme and α-amidation restoring factor were included, was also found to require ascorbate, copper ion, catalase and molecular oxygen. The optimal concentration of ascorbate, copper ion and catalase were 1.5 mM, 50 μM and 50 μg/ml respectively. The optimal amount of the α-amidation restoring factor prepared from tissue homogenate was 10-50% of final reaction volume.

Under the condition, wherein a half final volume of the restoring factor was added, the α-amidation activity reached about 70% of the original level.

The partially purified α-amidating enzyme used in the present invention was able to be prepared from rat brain or pituitary homogenate by chromatograph on a Sephacryl S-200 column. The α-amidating enzyme has an apparent molecular weight of 56,000 daltons. This was close to the 54,000 daltons reported for one of the purified bovine pituitary enzymes (J. Biol. Chem. 216, 1815-1822, 1986). The partially purified α-amidating enzyme had an extremely low activity and required ascorbate, copper ion and molecular oxygen for its maximum activity. From these observation, the α-amidating enzyme prepared from rat brain had properties similar to the activities found in other tissues reported by others. The present inventors also confirmed that the enzyme prepared from rat pituitary had similar properties.

As described above, the inevitable loss of α-amidating activities through purification was restored by addition of factor prepared from tissue homogenate. Some enzymatic cofactor could replace the factor prepared from tissue homogenate. Among cofactors tested, only the reaction system supplemented with pyrroloquinolinequinone in place of the factor showed significant activity. The maximum activity was attained at 0.1 mM of pyrroloquinolinequinone, but it was rather inhibitory when the concentration of pyrroloquinolinequinone was over 0.1 mM.

The pyrroloquinolinequinone-dependent restoring system also required copper ion, catalase and molecular oxygen but not ascorbate. In this restoring system, the optimal concentration of copper ion and catalase was 50 mM and 50 μg/ml respectively.

By applying the present invention, peptides con taining C-terminal glycine can be efficiently converted to des-glycine α-amide peptides.

More particularly the precursor molecules of peptide hormones or neuropeptides can be matured or modified by the present invention.

Furthermore, this invention can be applied to the maturation or modification of the peptide hormone- and neuropeptide-precursors produced by DNA recombinant technics, cell culture technics and chemical procedures.

According to the present invention, peptide hormone precursors and neuropeptide precursors, having C-terminal glycine, can be matured or modified to des-glycine α-amide peptides by cleavage of N-C bond in glycine molecule biologically, efficiently and cheaply. In particular, peptide hormone precursors and neuropeptide precursors, which were produced by DNA recombinant technics or cell culture technics, can be modified to mature peptides biologically, efficiently and cheaply.

Example 1

Preparation of Boiled Supernatant

Six rats (Wister strain) were killed by decapitation. Pooled brains were homogenized in a Potter-Elvehjem type glass homogenizer with a Teflon pestle in 0.25 M sucrose containing 50 mM Hepes•KOH (pH 7.0), 10 mM mannitol and 300 μg/ml of PMSF. The homogenate was certrifuged at 600 x g for 10 minutes. The supernatant was subjected to three cycles of freeze and thaw, followed by centrifugation at 20,000 x g for 10 minutes. The resulting supernatant was fractionated with solid ammonium sulfate between 0.2 and 0.5 saturation and the precipitate obtained was suspended in homogenizing buffer and then dialyzed overnight against 50 mM Tris•HCl buffer (pH 7.0) containing 30 μg/ml of PMSF. Insoluble materials were removed by centrifugation at 10,000 x g for 10 minutes. This fraction was referred to as ammonium sulfate fraction. An aliquot (1 to 2 ml) of the ammonium sulfate fraction was boiled for 5 minutes in a waterbath, cooled on ice followed by centrifugation at 10,000 x g for 10 minutes. The clear supernatant thus obtained was used for the restoring system as the boiled supernatant.

Example 2

Amidation Assay and Protein Determination

Amidation assays were performed according to the method of Glembotski et al. (J. Biol. Chem. 259, 6385-6392, 1984) with slight modification. Assay tubes (3 ml Eiken #2 tube, Eiken Tokyo) in duplicate contained in a final volume of 80 μl; mono- $^{125}$I-D-Try-Val-Gly (20,000-30,000 cpm), 2 μM of cold D-Try-Val-Gly, 1.5 mM of ascorbate, 50 μM of CuSO₄, 50 μg/ml of catalase, 50 mM of Hepes•KOH (pH 7.0) and appropriate amount of enzyme. The reaction was carried out at 37°C for 5 hours and terminated by the addition of 1 ml of cooled 2 mM sodium phosphate (pH 5.0). The diluted sample was then applied to a 2 ml column (Econo Column, Bio-Rad) of SP-Sephadex C-50 equilibrated with 2 mM of sodium phosphate (pH 5.0). The washings were combined (substrate fraction, 10.8 ml). The product was eluted sequentially with a 0.5 ml and two 1 ml aliquot of 50 mM sodium phosphate containing 0.5 M NaCl (product fraction, 3.7 ml). Each 1 ml aliquot of substrate and product fraction was counted in an Aloka autowell ganma system (model ARC-600) with a efficiency of about 80%. All radioactivity data were corrected for enzyme-free controls which had been subjected to the same α-amidation assay protocol. The reaction velocities were calculated as previously described (J. Biol. Chem 259, 6385-6392, 1984). Results were represented as the average of duplicate assays which agreed within 7%. The radioactivities in the product fractions were all analyzed by the RP-HPLC, and identified as mono-$^{125}$I-D-Try-Val-NH$_2$. Protein was determined by Lowry's method (J. Biol. Chem. 193, 265-275, 1951) using bovine serum albumin as standard.

Example 3

Isolation and partial purification of α-amidating enzyme

Six rats were killed by decapitation. Pooled brains were homogenized in a Potter-Elvehjem type glass homogenizer with a Teflon pestle in 0.25 M sucrose containing 50 mM Hepes•KOH (pH 7.0), 10 mM mannitol and 300 μg/ml of PMSF. The homogenate was certrifuged at 600 x g for 10 minutes. The supernatant was subjected to three cycles of freeze and thaw, followed by centrifugation of 20,000 x g for 10 minutes. The resulting supernatant was fractionated with solid ammonium sulfate between 0.2 and 0.5 saturation and precipitate obtained was suspended in homogenizing buffer and then dialyzed overnight against 50 mM Tris•HCl buffer (pH 7.0) containing 30 μg/ml of PMSF. Insoluble materials were removed by centrifugation at 10,000 x g for 10 minutes. (ammonium sulfate fraction). Onto a column of Sephacryl S-200 (1.5 x 45 cm) equilibrated with 10 mM Hepes•KOH (pH 7.0) containing 50 mM NaCl, 1.5 ml of aliquot of ammonium sulfate fraction (3.2 mg as protein) was applied and eluted with the same buffer at a flow rate of 5 ml/hr. Fractions of 0.92 ml were collected. The α-amidating activities were assayed using 25 μl of each fraction under the conditions described in example 2. Next, the enzyme activity was assayed with addition of 10 μl of the boiled supernatant prepared from ammonium sulfate fraction described in example 1.

As shown in Fig 1, the α-amidating activity was eluted as a single peak at fractions 48-53.

Example 4

Effect of boiled supernatant on α-amidating activity

Fractions 48-53 were combined and the α-amidating activity was assayed with the various amount of the boiled supernatant under the same conditions described in example 2 except the final volume ( 160 μl in place of 80 μl). Fig 2 shows a saturation curve of the α-amidating activity of the combined Sephacryl S-200 fraction with increasing amount of the boiled supernatant.

Example 5

Requirements for the restored α-amidating Activity

Requirements of the restored α-amidating activity was examined using 3.8 μg of combined Sephacryl S-200 fraction. As shown in Table, the restored system was found to require ascorbate, copper ion and molecular oxygen for its maximum activity. A half maximum activity was detected in the absence of ascorbate and almost no activity was detected by the omission of copper ion or oxgen.

| Exp. No. | Reaction system | D-Tyr-Val-NH$_2$ found pmol/mg/h |
|---|---|---|
| 1 | Restored with 10 $\mu$l of boiled supernatant | 1175 |
| 2 | Minus boiled supernatant | 35 |
| 3 | ascorbate | 665 |
| 4 | Cu$^{2+}$ | 26 |
| 5 | O$_2$ | 15 |
| 6 | catalase | 21 |

Example 6

The $\alpha$-amidating activities of the Pyrroloquinolinequinone (PQQ)-supplemented reaction system with or without ascorbate

The $\alpha$-amidating activity was assayed using 3.5 $\mu$g of the combined Sephacryl S-200 fraction under the same condition described in example 2.

| Exp. No. | Reaction system | Addition of ascorbate (1.5 mM) | D-Tyr-Val-NH$_2$ found pmol/mg/h |
|---|---|---|---|
| 1 | Restored with 10 $\mu$l of boiled supernatant | + | 1170 |
| | | - | 655 |
| 2 | Minus boiled supernatant and plus PQQ (0.1 mM) | + | 436 |
| | | - | 470 |
| 3 | Restored with 10 $\mu$l of boiled supernatant and plus PQQ (0.1 mM) | + | 766 |
| | | - | 863 |

Example 7

Ability of Pyrroloquinolinequinone (PQQ) to restore the $\alpha$-amidating activity

The ability of the boiled supernatant and PQQ to restore the $\alpha$-amidating activity was examined using 3.7 $\mu$g of the combined Sephacryl S-200 fraction under the same condition described in Example 2. As shown in Table, 0.1 mM of PQQ was able to restore the $\alpha$-amidating activity to about 40% of that obtained in the restored reaction system with the boiled supernatant.

| Exp. No. | Reaction system | D-Tyr-Val-NH$_2$ found pmol/mg/h |
|---|---|---|
| 1 | Restored with boiled supernatant | 1410 |
| 2 | Minus boiled supernatant | 51 |
| 3 | Minus boiled supernatnat plus PQQ (0.1 mM) | 490 |

**Claims**

1. A process which matures or modifies a peptide having C-terminal glycine to des-glycine α-amide peptide by cleavage of N-C bond of glycine in the presence of peptidyl-glycine α-amidating enzyme and either a biochemical catalyst prepared from tissue homogenate or an enzymatic cofactor.

2. The process as claimed in claim 1, wherein the peptide having C-terminal glycine is a peptide hormone precursor.

3. The process as claimed in claim 1, wherein the peptide having C-terminal glycine is a neuropeptide precursor.

4. The process as claimed in claim 1, wherein the biochemical catalyst derived from tissue homogenate is a fraction partially purified from ammonium sulfate precipitate fraction.

5. The process as claimed in claim 1, wherein the biochemical catalyst derived from tissue homogenate is a boiled supernatant prepared from ammonium sulfate precipitate fraction.

6. The process as claimed in claim 1, wherein the enzymatic cofactor is pyrroloquinolinequinone.

7

Fig. 1

Fig. 2